# EUROPÄISCHE PATENTANMELDUNG

(11) **EP 2 883 859 A1**
(43) Veröffentlichungstag der Anmeldung: **17.06.2015**
(21) Anmeldenummer: 13196810.9
(22) Anmeldetag: 12.12.2013
(51) Int. Cl.: C07C 45/75, C07C 47/22

(54) **Tertiäre Alkylamine als Cokatalysator bei der Methacrolein-Synthese**

(71) Anmelder: Evonik Industries AG, 45128 Essen (DE)
(72) Erfinder: Burghardt, Rudolf, Dr., 64287 Darmstadt (DE); Krill, Steffen, Dr., 64367 Mühltal (DE); Balduf, Torsten, Dr., 64319 Pfungstadt (DE); Rundal, Eduard, 60486 Frankfurt (DE)

(57) **Zusammenfassung**

Die vorliegende Erfindung betrifft ein Verfahren zur Herstellung von Methacrolein aus Propionaldehyd und Formalin. Dieses neuartige Verfahren zeichnet sich dadurch aus, dass das zu dieser Umsetzung verwendete Katalysatorsystem durch das gleichzeitige Vorliegen von sekundären und tertiären Aminen derart modifiziert wird, dass die Synthese effizienter und mit weniger Reinigungsaufwand durchgeführt werden kann.

## Beschreibung

Die vorliegende Erfindung betrifft ein verbessertes Verfahren zur Herstellung von Methacrolein aus Propionaldehyd und Formalin. Dieses neuartige Verfahren zeichnet sich dadurch aus, dass das zu dieser Umsetzung verwendete Katalysatorsystem derart modifiziert wird, dass die Synthese effizienter und mit weniger Reinigungsaufwand durchgeführt werden kann.

Methacrolein wird in der chemischen Synthese insbesondere als Zwischenprodukt zur Herstellung von Methacrylsäure, Methylmethacrylat oder auch von Wirk-, Geruchs- oder Geschmacksstoffen verwendet. Es besteht ein großes Interesse an möglichst einfachen, wirtschaftlichen und umweltschonenden Herstellungsverfahren.

### Stand der Technik

Die vorliegende Erfindung betrifft die Modifikation des großtechnisch bedeutenden Verfahrens zur Herstellung von Methacrolein, ausgehend von Propanal und Formaldehyd bzw. Formalin. Die Reaktion erfolgt über eine Mannich-Reaktion. Ein solches Verfahren zur Herstellung von Methacrolein, ist unter anderem in den Druckschriften DE 32 13 681, US 7,141,702, US 4,408,079, JP 3069420, JP 4173757, EP 0 317 909 und US 2,848,499 beschrieben.

Bei diesem Verfahren wird in Gegenwart von Dimethylamin und Essigsaure als homogene Katalysatoren bei Temperaturen von ca. 160 bis 180 °C und einer Verweilzeit von ca. 10 sec Propionaldehyd mit Formalin umgesetzt. Das Reaktionsgemisch wird destillativ getrennt. Am Kopf der Kolonne wird ein Methacrolein-Wasser Heteroazeotrop gewonnen. Am Sumpf der Kolonne wird eine wässrige Lösung mit den homogenen Katalysatoren, Spuren von Methacrolein und Hochsiedern abgetrennt. Optional kann ein Teil, beispielsweise ca. 50%, des wässrigen Austrages wieder recycliert werden.

Die für die Reaktion benötigten, katalytisch aktiven Amine bilden unter den Reaktionsbedingungen nach Eschweiler-Clarke bzw. in einer Leukart-Wallach ähnlichen Reaktion höher alkylierte Derivate, die gemäß Stand der Technik nicht mehr katalytisch aktiv sind. So bildet sich beispielhaft aus Dimethylamin durch Reaktion mit einem Formaldehyd-Equivalent Trimethylamin, während Wasser freigesetzt wird. Nach DE 32 13 681 ist dieses bei der Umsetzung aus Dimethylamin (DMA) gebildete Trimethylamin (TMA) katalytisch inaktiv. In US 4,408,079 wird zusätzlich ausgeführt, dass Trimethylamin die Selektivität der Umsetzung herabsetzt und daher abgetrennt werden muss. Bei einer solchen Abtrennung ist in einem kontinuierlichen Betrieb jedoch ein insgesamt höherer Katalysatorverbrauch gegeben. So ist das TMA nicht ohne gleichzeitige Abtrennung von DMA zu entfernen und es müssen sowohl die zu viel abgetrennten DMA-Anteile als auch das zu TMA umgesetzte DMA erneuert werden. Weiterhin erfolgt diese Abtrennung destillativ, wodurch auch das mit abgetrennte niedrigsiedende Methacrolein in einer aufwendigen weiteren Destillationsstufe zurück gewonnen werden muss.

Nach dem Stand der Technik ist es somit erforderlich, bei Recyclierung des Sumpfstromes die Zugabe von DMA zu erhöhen, um den Verlust an katalytischer Aktivität infolge der Bildung von TMA auszugleichen.

### Aufgabe

In Anbetracht des Standes der Technik war es daher Aufgabe der vorliegenden Erfindung, die während der Durchführung einer kontinuierlich durchgeführten, aminkatalytischen Reaktion zur Synthese von Methacrolein eingesetzte Menge an Aminen gegenüber dem Stand der Technik zu reduzieren.

Insbesondere war es Aufgabe, ein solches Verfahren zur Verfügung zu stellen, bei dem weniger Reinigungsschritte benötigt werden und die Abwassermenge reduziert werden kann.

Weitere nicht explizit genannte Aufgaben ergeben sich aus dem Gesamtzusammenhang der nachfolgenden Beschreibung und der Ansprüche.

### Lösung

Das erfindungsgemäße Verfahren wird zur Optimierung der aminhaltigen Komponenten des Katalysatorsystems einer - bevorzugt kontinuierlich durchgeführten - Mannich-Reaktion, bei der Propanal mit Formaldehyd zu Methacrolein umgesetzt wird, eingesetzt.

Gelöst werden diese Aufgaben durch ein neuartiges Verfahren bei dem Propanal in Gegenwart von 0,1 bis 30 Mol%, bevorzugt 0,5 bis 20 Mol% organische Base und 0,1 bis 30 Mol%, bevorzugt 0,5 bis 20 Mol% Säure, jeweils bezogen auf Propanal, bei einer Temperatur von 100 bis 300 °C und bei einem Druck von 5 bis 100 bar kontinuierlich mit Formaldehyd zu Methacrolein umgesetzt wird. Dabei ist die Komponente Formaldehyd unabhängig von der Zugabeform zu verstehen und Formalin oder Paraformaldehyd sind mit diesem Begriff in Bezug auf die Zugabe mit umfasst.

Das erfindungsgemäße Verfahren zeichnet sich insbesondere dadurch aus, dass es sich bei der organischen Base um eine Mischung aus einem sekundären Alkylamin und einem tertiären Alkylamin in einem Stoffverhältnis zwischen 20 zu 1 und 1 zu 3, bevorzugt zwischen 10 zu 1 und 1 zu 2, besonders bevorzugt zwischen 7 zu 1 und 1 zu 1 und ganz besonders bevorzugt zwischen 5 zu 1 und 2,5 zu 1 handelt.

In einer ersten Ausführungsform werden die sekundären und die tertiären Alkylamine der Anlage, bevorzugt direkt dem Reaktor, als Mischung zugegeben. Dabei ist das Verhältnis der beiden Komponenten entweder wie angegeben oder der Anteil der tertiären Alkylamine am Eingang der Reaktion ist vermindert gegenüber dem Reaktorausgang, da diese während der Reaktion von sich aus gebildet werden.

In einer bevorzugten, alternativen Ausführungsform der Erfindung werden der Anlage nur sekundäre Alkylamine zugegeben. Die tertiären Alkylamine werden dabei während des Betriebs der Anlage gebildet und reichern sich bei Kreislaufführung an. Besonders bevorzugt wird in dieser Ausführungsform der Gehalt an tertiären Alkylaminen kontinuierlich oder zum Beispiel durch Probenentnahme regelmäßig bestimmt. Auf Basis dieser Bestimmung wird die aminhaltige Katalysatorlösung bei Unterschreiten des Verhältnisses von sekundären zu tertiären Aminkomponenten unter 1 zu 3, bevorzugt unter 1 zu 2 und besonders bevorzugt unter 1 zu 1 entsprechend erneuert. Diese Erneuerung erfolgt durch Ausschleusen der wässrigen Reaktionsphase und/oder durch Zugabe frischen sekundären Alkylamins zum System, bevorzugt direkt in den Reaktor. Dazu ist die Anlage derart gestaltet, dass die Reaktionsmischung kontinuierlich aus dem Reaktionsraum ausgeschleust wird und über einen Phasentrenner oder bevorzugt über eine Destillationskolonne getrennt wird. Dabei wird die organische, das Methacrolein enthaltene Phase zum Beispiel als Destillat gewonnen und abgeführt. Der zurückbleibende wässrige Destillationssumpf, unter anderem auch Reste des Methacroleins enthaltend, wird ganz oder teilweise in den Reaktionsraum zurückgeführt. An dieser Stelle können zur Erneuerung der Katalysatorkomponenten, insbesondere der Aminkomponenten, Teile der wässrigen Phase entnommen werden. Die Entnahme über einen Phasentrenner erfolgt analog. Durch die Entnahme erfolgt dabei nicht nur die Erneuerung der Katalysatorkomponenten, sondern es werden gleichzeitig die Wassermenge reduziert und entstandene Nebenprodukte werden aus dem System egeschleust.

Die Zuführung der aufzuarbeitenden Reaktionslösung aus dem Reaktionsraum in die Destillationskolonne kann dabei oberhalb, innerhalb oder unterhalb der Trennsektion der Kolonne erfolgen. Im Verdampferteil dieser Kolonne sammelt sich ein Sumpf, der überwiegend aus Reaktionswasser besteht bzw. die Wassermengen enthält, die mit der Formalinlösung in den Kreislaufprozess gelangen. Dieser Sumpf enthält zusätzlich die Katalysatorkomponenten, wie zum Beispiel die organische Säure und das sekundäre sowie das tertiäre Amin bzw. die aus diesen gebildeten Salze, sowie Nebenprodukte der Reaktion oder aus der Kombination aus beiden. Diese wässrige Katalysatorlösung kann bevorzugt unterhalb des Zulaufs zur Kolonne am Sumpf der Kolonne abgezogen werden. Das vorliegende Gesamtwasser setzt sich dabei aus dem Wasser, welches als Katalysatorlösung zugegeben wurde, dem bei der Reaktion gebildeten und optional dem aus der Formaldehydlösung zusammen. Weitere, jedoch in geringerem Umfang zu berücksichtigende Wasserquellen sind Bestandteile der technischen Edukte wie Propional und Wasser, das in diversen Nebenreaktionen der Katalysatorkomponten mit Edukten, Nebenprodukten und Reaktionsprodukten gebildet wird, sowie Reaktionswasser aus allen diesen Komponenten, die unter den Reaktionsbedingungen entstehen.

Außerdem ist es möglich, den Sumpfablauf so in zwei Teilströme aufzuteilen, dass ein Teilstrom gerade die Menge an Wasser mit sich führt, die bei der Reaktion gebildet bzw. mit den Ausgangsstoffen eingegeben wurde. Dieser Teilstrom wird dann ausgeschleust und der restliche Anteil in den Reaktor zurückgeführt. Der ausgeführte Teil würde bei diesem Vorgehen beispielsweise thermisch entsorgt oder biologisch aufgearbeitet.

Die auf diese Weise kontinuierlich, semikontinuierlich oder batchweise entnommene wässrige Phase der Reaktion wird darauf bevorzugt abgeführt, optional über eine Membran in zwei Phasen getrennt und das so gewonnene aminhaltige Abwasser (Retentat) in einem Thermal Oxidizers verbrannt. Vor dem Verbrennen kann das Abwasser über eine zusätzliche Destillation noch weiter aufgetrennt werden, um auf diese Weise z.B. mit abgeführtes Produkt zurück in den Reaktionskreislauf oder die Produktaufarbeitung zu leiten. Das Retentat der Membrantrennstufe kann ganz oder teilweise zurück in den Reaktor oder die Aufarbeitung geführt werden. Insbesondere bevorzugt wird die wässrige Phase der Reaktion abgeführt und über eine Membran in zwei Phasen getrennt. Das so gewonnene aminhaltige Abwasser wird dann in einem Thermal Oxidizers verbrannt oder anderweitig, z.B. in einer biologischen Aufarbeitung, entsorgt und das Retentat der Membrantrennung wird teilweise in den Reaktor zurückgeführt. Zusätzlich ist es möglich, das Retentat in einer Destillationsstufe weiter aufzukonzentrieren. Das so gewonnene Konzentrat kann dann teilweise zurück in den Reaktor geführt werden.

Alternativ zu einer Membrantrennung kann die wässrige Phase der Reaktion abgeführt werden und in einer Destillation getrennt werden. Der so gewonnene Destillationssumpf wird dann zurück in den Reaktor geführt. Die wässrige Phase wird zur Entsorgung abgeführt. Bei dieser Entsorgung kann es sich beispielsweise um die Verbrennung in einem Thermal Oxidizer oder eine biologische Aufarbeitung handeln.

Das gewonnene Methacrolein wiederum wird insbesondere bevorzugt nach einer Aufreinigung in einem kontinuierlich an das Verfahren anschließenden zweiten Verfahren zu Methacrylsäure oder Methylmethacrylat umgesetzt.

Die zur Herstellung von Methacrolein geeigneten, auf einer Mannich-Reaktion basierenden Verfahren sind dem Fachmann bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2. Insbesondere bezieht sich das erfindungsgemäß vor der Zuleitung des aminhaltigen Reaktionswassers besonders bevorzugt durchgeführte Verfahren auf eine kontinuierlich durchgeführte Mannich-Reaktion, wie sie in der europäischen Patentanmeldung mit dem Anmeldeaktenzeichen 13002076.1 offenbart ist. Erfindungsgemäß wird zur Verdeutlichung einer solchen bevorzugten Vorstufe auf den Offenbarungsgehalt dieser Anmeldung bezüglich der Methacrolein-Synthese Bezug genommen.

Eine solche, besonders bevorzugte Mannich-Reaktion wird in Gegenwart von 0,1 bis 20 Mol% organische Base, bevorzugt einem sekundären Amin, und 0,1 bis 20 Mol% Säure, jeweils bezogen auf das eingesetzte Propanal, bei einer Temperatur von 100 bis 300 °C und bei einem Druck von 5 bis 100 bar durchgeführt.

Bei den Säuren handelt es sich in der Regel um anorganische Säuren oder organische Mono-, Di- bzw. Polycarbonsäuren, bevorzugt Monocarbonsäuren, insbesondere aliphatische Monocarbonsäuren. Besonders bevorzugt wird zur Umsetzung von Propanal und Formaldehyd mindestens eine organische Säure, besonders bevorzugt Essigsäure eingesetzt. Der Anteil an Säure beträgt zwischen 0,1 und 20, vorteilhaft von 0,5 bis 10, bevorzugt 1 bis 5 Mol%, bezogen auf Propanal.

Als anorganische Säuren kommen insbesondere Schwefelsäure, Phosphorsäure, Salzsäure, Kohlensäure oder Borsäure in Frage. Als organische kommen neben Essigsäure auch Ameisensäure, Propionsäure, iso-Buttersäure, n-Buttersäure, Oxalsäure, Adipinsäure, Caprylsäure, Phenylameisensäure und 2-Ethyl-hexylsäure in Frage. Insbesondere können auch Gemische aus zwei oder mehreren Säuren in Frage kommen. Dabei sind auch Gemische aus organischen und anorganischen Säuren, insbesondere aus Essigsäure und Schwefelsäure gut geeignet.

Bevorzugt wird eine organische Säure oder eine Mischung aus einer organischen Säure und Schwefelsäure verwendet. Besonders bevorzugt wird nur Ameisensäure, Essigsäure oder Propionsäure eingesetzt.

Als sekundäre Amine, die als Katalysator dem System zugeführt werden, kommen beispielsweise in Betracht: Dimethylamin, Diethylamin, Methylethylamin, Methylpropylamin, Dipropylamin, Dibutylamin, Di-iso-propylamin, Di-iso-butylamin, Methyl-iso-propylamin, Methyl-iso-butylamin, Methyl- sek.-butylamin, Methyl-(2-methylpentyl)-amin, Methyl-(2-ethylhexyl)-amin, Pyrrolidin, Piperidin, Morpholin, N-Methylpiperazin, N-Hydroxyethyl-piperazin, Piperazin, Hexamethylenimin, Diethanolamin, Methylethanolamin, Methylcyclohexylamin, Methylcyclopentalamin, Dicyclohexylamin oder entsprechende Gemische. Bevorzugt handelt es sich bei dem sekundären Amin um Dimethylamin oder Diethylamin, besonders bevorzugt um Dimethylamin.

Als tertiäre Amine, die als Katalysator dem System zugeführt werden oder während des Verfahrens gebildet werden, kommen beispielsweise in Betracht: Trimethylamin, Triethylamin, Dimethylethylamin, Diethylmethylamin Dimethylpropylamin, Dipropylmethylamin, Tripropylamin, Tributylamin, Tri-iso-propylamin, Tri-iso-butylamin, Dimethyl-iso-propylamin, Dil-iso-propylmethylamin, Dimethyl-iso-butylamin, Di-iso-butylmethylamin, Triethanolamin, Dimethylethanolamin, Diethanolmethylamin, Dimethylcyclohexylamin, Dicyclohexylmethylamin, Dimethylcyclopentalamin, Dicyclopentalmethylamin, Triicyclohexylamin oder entsprechende Gemische. Bevorzugt handelt es sich bei dem tertiären Amin um Trimethylamin oder Triethylamin, besonders bevorzugt um Trimethylamin.

Ganz besonders bevorzugt handelt es sich bei dem sekundären Alkylamin um Dimethylamin und gleichzeitig bei dem tertiären Alkylamin um Trimethylamin.

Der Anteil an organischer Base beträgt zwischen 0,1 und 30, vorteilhaft von 0,5 bis 20, bevorzugt 1 bis 10 Mol%, bezogen auf Propanal.

Das Verhältnis der Äquivalente Amin zu Säure wird vorzugsweise so gewählt, dass im Reaktionsgemisch vor der Reaktion ein pH-Wert von 2,5 bis 9 resultiert.

Insbesondere ist es ein großer Vorteil der vorliegenden Erfindung, dass die MAL-Synthese gegenüber dem Stand der Technik mit deutlich reduzierter Abwassermenge durchgeführt werden kann. Da das tertiäre Alkylamin überraschend nicht kontinuierlich aus dem System ausgeschleust und gleichzeitig das sekundäre Amin in geringerem Maße erneuert werden muss, lässt sich der kontinuierliche Produktionsprozess mit dieser verringerten Abwassermenge durchführen.

Damit reduziert sich als weiterer Vorteil gleichzeitig die Zahl der Reinigungsschritte. Dies gilt insbesondere für das Abwasser, dass beim Ausschleusen deutlich konzentrierter vorliegt.

Ein großer Vorteil des erfindungsgemäßen Verfahrens ist, dass dieses mit relativ einfachen und kostengünstigen bzw. in bereits bestehenden Anlagen durchgeführt werden kann. Hierbei sind die Anlagen einfach zu warten und verursachen geringe Unterhaltskosten.

Die nachfolgenden Beispiele dienen zur näheren Erläuterung bevorzugter Ausführungsformen der vorliegenden Erfindung, ohne dass hierdurch eine Begrenzung der Erfindung erfolgen soll.

### Beispiele

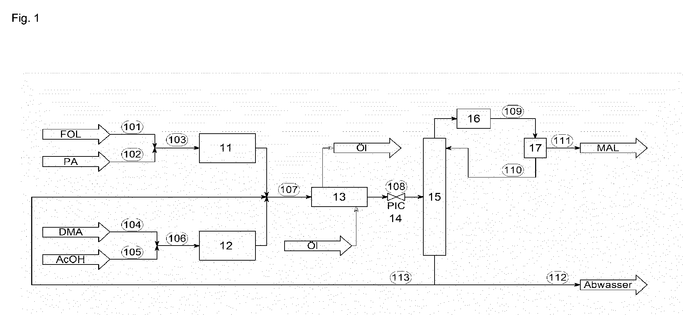

In einer Anlage entsprechend Fig. 1 wird kontinuierlich Propanal (PA) mit Formaldehyd unter Verwendung von Dimethylamin (DMA) bzw. einem DMA-TMA-Gemisch und einer Mischung aus Essigsäure (AcOH) zu Schwefelsäure von 2,8 zu 1 umgesetzt. Dabei wurde das Amin-Protonen-Verhältnis auf 0,75 eingestellt. Bei der Berechnung dieses Verhältnisses wird die Schwefelsäure gegenüber der Essigsäure naturgemäß doppelt berücksichtigt. Die eingesetzten Mengen Amin gehen aus Tabelle 1 hervor.

251 g/h PA und 349 g/h eines 37-prozentigen Formalin werden homogen vorgemischt (Verhältnis molar 1:1). Die Katalysatorlösung, bestehend aus den genannten Aminen und Säuren, wird in den Vorheizer (12) gefahren. Beide Ströme werden vor Vereinigung auf eine Temperatur von 170 °C erhitzt. Die vorerhitzten Ströme werden in einem T-Mischer vereinigt, der direkt mit einem Strömungsrohrreaktor (1/16 Zoll Rohr mit einer Länge von 4,2 m) verbunden ist. Die Thermostatisierung des Reaktors erfolgt mit einem Ölbad, das bei 180 °C betrieben wird, die Verweilzeit beträgt unter 10 s (siehe Tabelle 1), der Druck im Rohrreaktor 70 bar. Nach dem Rohrreaktor wird das Gemisch in Ventil (14) entspannt und der Kolonne (15) zugeführt. 335 g/h des Sumpfablaufes werden in den Reaktor (13) zurückgeführt, 370 g/h des Sumpfablaufes als Abwasser entsorgt. Nach Verflüssigung des Kopfstroms im Kondensator (16) und Phasentrennung in (17) wird eine methacroleinreiche Phase mit einem Methacrolein-Gehalt von 96,5 % als Produkt (111) abgeführt und der wässrige Ablauf des Phasentrenners in die Kolonne (15) zurückgeführt. Der Umsatz des Propionaldehyds, sowie die Ausbeute, bezogen auf das eingesetzte Propionaldehyd sind in Tabelle 1 dargestellt.

Die Reaktionen der Beispiele 3 bis 5, sowie die Vergleichsbeispiele (VB) 4 und 5 wurden abweichend bei einer Ölbadtemperatur von 160 °C, anstelle von 180 °C durchgeführt. Darüber hinaus wurde bei diesen Beispielen / Vergleichsbeispielen als Säure ausschließlich Essigsäue und kein Gemisch aus Schwefelsäure und Essigsäure verwendet. Dabei wurde ein Amin-Protonen-Verhältnis von 0,91 eingestellt.

**Tabelle 1**

| | Menge DMA mol / mol PA | Menge TMA mol / mol PA | Verweilzeit /s | Umsatz PA / % | Ausbeute MAL /% |
|---|---|---|---|---|---|
| Bsp.1 | 0,029 | 0,011 | 8,6 | 99,8 | 95,6 |
| Bsp.2 | 0,022 | 0,018 | 8,6 | 91,4 | 96,7 |
| VB1 | 0,040 | 0 | 8,6 | 99,9 | 95,4 |
| VB2 | 0,029 | 0 | 8,6 | 98,7 | 96,7 |
| VB3 | 0,019 | 0 | 8,6 | 82,7 | 92,7 |
| Bsp.3 | 0,039 | 0,0068 | 9,5 | 99,2 | 97,0 |
| Bsp.4 | 0,039 | 0,0136 | 9,5 | 99,5 | 97,0 |
| VB4 | 0,039 | 0 | 9,5 | 98,4 | 96,5 |
| Bsp.5 | 0,061 | 0,047 | 9,5 | 99,9 | 98,1 |
| VB5 | 0,060 | 0 | 9,5 | 99,9 | 98,0 |
| Bsp.6 | 0,062 | 0,024 | 9,7 | 99,9 | 98,0 |
| Bsp.7 | 0,064 | 0,064 | 9,4 | 99,9 | 98,0 |
| Bsp.8 | 0,065 | 0,084 | 9,3 | 99,9 | 98,0 |
| Bsp.9 | 0,080 | 0,160 | 9,5 | 99,9 | 98,0 |
| Bsp. 10 | 0,064 | 0,127 | 9,7 | 99,9 | 97,6 |
| VB6 | 0,042 | 0 | 9,5 | 98,3 | 96,5 |
| Bsp.11 | 0,042 | 0,007 | 9,5 | 99,6 | 97,5 |
| Bsp.12 | 0,042 | 0,015 | 9,5 | 99,8 | 97,5 |
| Bsp.13 | 0,042 | 0,022 | 9,5 | 99,8 | 97,5 |

Aus den Beispielen 1 und 2 geht im Vergleich zu den Beispielen VB2 und VB3 klar hervor, dass bei zusätzlichem Vorliegen von TMA, bei identischen DMA-Mengen die Ausbeute und der Umsatz deutlich gesteigert werden können. Aus dem Vergleich der Bsp.1 und VB1 wird sogar das gegenüber dem Stand der Technik sehr überraschende Ergebnis sichtbar, dass bei kleineren TMA-Gehalten von unter 40 mol% und einer identischen Amin-Stoffkonzentration sowohl die Ausbeute als auch der Propionaldehyd-Umsatz fast unverändert sind. Erst bei höheren Anteilen an TMA, entsprechend Beispiel 2, gehen beide etwas zurück. Jedoch sind auch in diesem Fall sowohl der Umsatz als auch die Ausbeute noch in einem wirtschaftlich interessanten Bereich.

Aus den Beispielen 3 und 4 geht wiederum in Hinblick auf Vergleichsbeispiel 4 hervor, dass auch bei einem Anreichern von TMA im Reaktor - wie es hier durch zusätzliche Mengen im Versuchsaufbau simuliert wird - überraschend zu höheren Umsätzen und Ausbeuten führt, auch wenn die Selektivität minimal reduziert zu werden scheint. Dieser kleine Selektivitätsverlust wird durch die Umsatzsteigerung jedoch mehr als ausgeglichen.

Beispiele 5 und 6 und Vergleichsbeispiel 5 zeigen wiederum, dass man mit besonders hohen Aminkonzentrationen erwartungsgemäß einen besonders hohen Umsatz und hohe MAL-Ausbeuten erzielen kann. Überraschend und im großen Widerspruch zum Stand der Technik ist jedoch, dass man diese hohen Umsätze auch dann erreicht, wenn derart viel Trimethylamin im Reaktor vorliegt, dass das Verhältnis von Trimethylamin zu Dimethylamin nur knapp unter 0,8 liegt.

Aus den Beispielen 7 bis 10 ist ersichtlich, dass man sogar bei äquimolaren Mengen TMA und DMA oder bei einem TMA-Überschuss überraschend hohe Ausbeuten und Selektivitäten erzielen kann. Dies zeigt zuletzt, dass mit vorliegender Erfindung ein lange bestehendes Vorurteil des Standes der Technik überwunden wurde.

Dem Fachmann ist leicht ersichtlich, dass diese Versuche auch auf kontinuierliche Herstellprozesse, bei denen sich TMA anreichert, übertragbar sind.

Aus den Beispielen VB6 sowie 11 bis 13 ist wiederum ersichtlich, dass bei konstanter DMA-Konzentration und konstanten anderen Prozessparametern bei gleichzeitig steigender TMA-Konzentration sowohl die Selektivität als auch die Ausbeute überraschend steigen. Daraus lässt sich nicht nur schließen, dass TMA entgegen der allgemeinen Lehre des Standes der Technik nicht bei der Reaktion stört, sondern selbst katalytisch zur Reaktion beiträgt.

### Bezugszeichenliste

### Fig. 1: Schematische Darstellung einer zur Durchführung des erfindungsgemäßen Verfahrens geeigneten Anlage

- FOL: Formalin (wässrige Formaldehyd-Lösung)
- PA: Propanal
- DMA: wässrige Dimethylamin-Lösung
- AcOH: Essigsäure
- MAL: Methacrolein
- 11: Wärmetauscher (Vorwämer)
- 12: Wärmetauscher (Vorwämer)
- 13: Reaktor (Rohrreaktor)
- 14: Druckhalteventil
- 15: MAL-Destillationskolonne
- 16: Kondensator
- 17: Phasentrenner
- 101: Leitung wässriges Formalin
- 102: Leitung Propanal
- 103: Leitung in Wärmetauscher
- 104: Leitung Dimethylamin (40%ige wässrige Lösung)
- 105: Leitung Essigsäure
- 106: Leitung in Wärmetauscher
- 107: Leitung in Reaktor
- 108: Leitung Produktgemisch zur Kolonne
- 109: Leitung Kondensat
- 110: Rückstrom in Kolonne
- 111: MAL zu Stufe B)
- 112: Leitung zu Abwasser
- 113: Rückführung Sumpfablauf

## Patentansprüche

**1.** Verfahren bei dem Propanal in Gegenwart von 0,1 bis 30 Mol% organische Base und 0,1 bis 30 Mol% Säure, jeweils bezogen auf Propanal, bei einer Temperatur von 100 bis 300 °C und bei einem Druck von 5 bis 100 bar kontinuierlich mit Formaldehyd zu Methacrolein umgesetzt wird, **dadurch gekennzeichnet, dass** es sich bei der organischen Base um eine Mischung aus einem sekundären Alkylamin und einem tertiären Alkylamin in einem Stoffverhältnis zwischen 20 zu 1 und 1 zu 3 handelt.

**2.** Verfahren gemäß Anspruch 1, **dadurch gekennzeichnet, dass** das Stoffverhältnis zwischen dem sekundärem Alkylamin und dem tertiären Alkylamin zwischen 7 zu 1 und 1 zu 1 liegt.

**3.** Verfahren gemäß Anspruch 1 oder 2, **dadurch gekennzeichnet, dass** das es sich bei dem sekundären Alkylamin um Dimethylamin und bei dem tertiären Alkylamin um Trimethylamin handelt.

**4.** Verfahren gemäß mindestens einem der Ansprüche 1 bis 3, **dadurch gekennzeichnet, dass** die Alkylamine der Anlage als Mischung zugegeben werden.

**5.** Verfahren gemäß mindestens einem der Ansprüche 1 bis 3, **dadurch gekennzeichnet, dass** der Anlage nur sekundäre Alkylamine zugegeben werden, sich die tertiären Alkylamine während des Betriebs der Anlage anreichern, das Verhältnis von sekundären zu tertiären Alkylaminen kontinuierlich oder regelmäßig bestimmt wird und die aminhaltige Katalysatorlösung bei Unterschreiten des Verhältnisses der Aminkomponenten von sekundären Alkylamin zu tertiären Alkylamin von 1 zu 3 erneuert wird.

**6.** Verfahren gemäß mindestens einem der Ansprüche 1 bis 5, **dadurch gekennzeichnet, dass** kontinuierlich, semikontinuierlich oder batchweise die wässrige Phase der Reaktion abgeführt wird, optional über eine Membran in zwei Phasen getrennt, das so gewonnene aminhaltige Abwasser in einem Thermal Oxidizers verbrannt und das Retentat der Membrantrennung ganz oder teilweise zurück in den Reaktor geführt wird.

**7.** Verfahren gemäß Anspruch 6, **dadurch gekennzeichnet, dass** die wässrige Phase der Reaktion abgeführt wird, über eine Membran in zwei Phasen getrennt, das so gewonnene aminhaltige Abwasser in einem Thermal Oxidizers verbrannt und das Retentat der Membrantrennung in einer Destillationsstufe konzentriert wird und das Konzentrat zurück in den Reaktor geführt wird.

**8.** Verfahren gemäß mindestens einem der Ansprüche 1 bis 5, **dadurch gekennzeichnet, dass** die wässrige Phase der Reaktion abgeführt wird, in einer Destillation getrennt wird, der Destillationssumpf zurück in den Reaktor geführt wird und die wässrige Phase zur Entsorgung abgeführt wird.

**8.** Verfahren gemäß mindestens einem der Ansprüche 1 bis 7, **dadurch gekennzeichnet, dass** das gewonnene Methacrolein in einem kontinuierlich an das Verfahren anschließenden zweiten Verfahren zu Methacrylsäure oder Methylmethacrylat umgesetzt wird.

**9.** Verfahren gemäß mindestens einem der Ansprüche 1 bis 8, **dadurch gekennzeichnet, dass** als Säure eine oder mehrere organische Säuren oder eine Mischung aus einer organischen Säure und Schwefelsäure verwendet werden.

**10.** Verfahren gemäß Anspruch 9, **dadurch gekennzeichnet, dass** als Säure Ameisensäure, Essigsäure oder Propionsäure verwendet wird.
